# EUROPEAN PATENT SPECIFICATION

(11) **EP 4 196 179 B1**
(45) Date of publication and mention of the grant of the patent: **11.12.2024**
(21) Application number: 21755841.0
(22) Date of filing: 11.08.2021
(51) Int. Cl.: A61L 2/14, H05H 1/24, F25D 17/06, H01R 4/00

(54) **PLASMA SOURCE FOR GENERATING A DISINFECTING AND/OR STERILIZING GAS MIXTURE**
PLASMAQUELLE ZUR ERZEUGUNG EINES DESINFIZIERENDEN UND/ODER STERILISIERENDEN GASGEMISCHES
SOURCE DE PLASMA POUR GÉNÉRER UN MÉLANGE DE GAZ DE DÉSINFECTION ET/OU DE STÉRILISATION

(30) Priority: 11.08.2020 NL 2026249
(43) Date of publication of application: 21.06.2023
(73) Proprietor: Log10 B.V., 5642 JC Eindhoven (NL)
(72) Inventor: VAN DER LEIJ, Theo Alex Eduard, 5642 JC Eindhoven (NL); VERHOEVEN, Franciscus Maria, 5642 JC Eindhoven (NL); PEETERS, Mirte, 5642 JC Eindhoven (NL); DE JONG, Thijs, 5642 JC Eindhoven (NL)
(74) Representative: V.O.
(86) International application number: PCT/NL2021/050505
(87) International publication number: WO 2022/035317

(56) References cited:
- EP-A1- 3 500 072
- WO-A1-2018/005715
- GB-A- 2 501 933
- GB-A- 2 526 627

## Description

### FIELD OF THE INVENTION

The present invention relates to disinfecting and/or sterilizing medical instruments, such as dental instruments using a plasma. More in general, the present invention relates to methods and devices for generating a disinfecting and/or sterilizing gas mixture.

### BACKGROUND TO THE INVENTION

Reusable medical instruments are instruments that health care providers can reuse to diagnose and/or treat multiple patients. Examples of reusable medical instruments include medical instruments used in dental care, such as scalpels, syringes, scopes, mirrors, drills, burs, discs, handpieces, excavators, turbines, files, reamers, etc.

When used on patients, reusable instruments become soiled and contaminated with blood, tissue and other biological debris such as microorganisms. To avoid any risk of infection by a contaminated instrument, the reusable instruments can be sterilized. Sterilizing results in a medical instrument that can be safely used more than once in the same patient, or in more than one patient. Adequate sterilizing of reusable medical instruments is vital to protecting patient safety.

Various sterilizing agents can be used for sterilizing medical instruments. Historically, steam or hydrogen peroxide is often used. More recently, plasma devices are being used for ionizing gases or gas mixtures, the ionized gas being used as sterilizing agent. Electrons in the plasma impact on gas molecules causing dissociation and ionization of these molecules, which creates a mix of reactive species. It is known to directly expose the medical instruments to the plasma, or to expose the medical instruments to the (partially) recombined plasma, sometimes referred to as afterglow, see e.g. S. Moreau et al., "Using the flowing afterglow of a plasma to inactivate Bacillus subtilis spores: Influence of the operating conditions", J. Appl. Phys. Vol. 88, No. 2, 15 July 2000.

Several attempts have been made to improve upon plasma sterilizing. US2011/0027125A1 discloses a system comprising a chamber and a plasma generator for generating free radicals combined with use of a hydrogen peroxide solution.

WO2018/005715A1 relates to a system and methods for sterilizing or disinfecting articles, particularly the hollow internal areas of medical instruments. The system includes a plasma generator having an electrode, a shield, and a dielectric gap between the electrode and the shield. A source of electrical power is connected to the plasma generator for applying an electron energy density between the electrode and the shield. A source of gas comprising water vapor, oxygen and nitrogen provides a flow of gas between the electrode and the shield, to form a plasma containing acidic and/or oxidizing species.

GB2501933A relates to a device for providing a flow of non-thermal gaseous plasma for treatment of a treatment region comprising a cell for the generation of the non-thermal plasma. The cell has an inlet for gas and an outlet for the non-thermal gaseous plasma. The cell is in a heat conducting relationship with a heat sink through a heat pipe, the heat sink and the heat pipe both forming parts of the device. The cell typically has a dielectric member of high thermal conductivity in heat conducting relationship with the heat pipe. The heat sink is typically a capsule containing the gas to be supplied to the inlet of the cell.

It is also known to use an atmospheric or super atmospheric plasma source.

Plasma sources can have the disadvantage that the composition of the disinfecting and/or sterilizing agent, produced by generating an at least partially ionized gas mixture, can vary significantly with varying temperature and/or pressure of the plasma.

### SUMMARY OF THE INVENTION

It is an object to provide an improved plasma source for generating a disinfecting and/or sterilizing gas mixture.

Thereto, according to a first aspect, is provided a plasma source for generating a disinfecting and/or sterilizing gas mixture. The plasma source includes an ionization chamber. The ionization chamber includes a dielectric tubular portion. The dielectric tubular portion can form a wall of the ionization chamber. The ionization chamber includes an inflow port for feeding a gas or gas mixture into the chamber. In the ionization chamber the gas or gas mixture is transformed into the disinfecting and/or sterilizing gas mixture. The ionization chamber includes an outflow port for exhausting the disinfecting and/or sterilizing gas mixture out of the chamber. The inflow port can be positioned at a first end of the tubular portion. The outflow port can be positioned at an opposite, second end of the tubular portion. Hence, a gas or gas mixture can be made to flow through the tubular portion. The ionization chamber includes a first electrode positioned inside the dielectric tubular portion, and a second electrode positioned outside the dielectric tubular portion. The first electrode can e.g. extend longitudinally within the tubular portion, such as along the axis of the tubular portion. The second electrode can be formed on an outer surface of the tubular portion. The second electrode can be a separate part, such as a metal sheet. It is also possible that the second electrode is a conductive layer coated onto the outer surface of the tubular portion, such as a metallic layer (plasma) deposited onto the outer surface. The plasma source includes a high voltage source having a high voltage output terminal, wherein an electrical conductor connects the output terminal to the first or second electrode. The high voltage terminal can e.g. be connected to the first electrode. The second electrode can be connected to electrical ground. The electrical conductor is preferably less than 50 cm long. The plasma source includes a forced gas cooling system for cooling the ionization chamber.

The forced gas cooling system is arranged for forcing a cooling gas flow onto the dielectric tubular portion in a direction substantially orthogonal to a longitudinal axis of the tubular portion, e.g. perpendicular to the longitudinal axis of the tubular portion. It has been found that such flow effectively provides high quality of the disinfecting and/or sterilizing gas mixture. The forced gas cooling system comprises an elongate funnel having two sidewalls tapering towards the dielectric tubular portion and extending along the longitudinal axis, wherein the funnel is arranged between at least one fan and the dielectric tubular portion and configured to force the cooling gas flow by the fan into the funnel towards its narrow end where the two sidewalls converge, wherein the narrow end has an elongate exit opening disposed adjacent the dielectric tubular portion and extending along the longitudinal axis, wherein the exit opening is arranged facing the dielectric tubular portion and configured to force the cooling gas flow exiting the opening to flow onto one side of the tubular portion in the direction substantially orthogonal to the longitudinal axis.

The plasma source is arranged for generating a disinfecting and/or sterilizing gas mixture. It will be appreciated that depending on the circumstances it may suffice to generate a disinfecting gas mixture suitable for disinfecting objects, wherein a large proportion of microorganisms is killed, although not all microorganisms are necessarily killed. In other cases it is preferred to generate a sterilizing gas mixture suitable for sterilizing an object, wherein substantially all microorganisms are killed.

It has been found that cooling the ionization chamber, e.g. the tubular portion of the ionization chamber, with forced gas, such as forced air, provides particular good disinfecting and/or sterilizing gas mixture, especially when combined with the relatively short electrical conductor.

It is thought that the forced gas cooling improves quality of the disinfecting and/or sterilizing gas mixture by beneficially influencing temperature stability of the plasma source. In this respect it has been found that gas cooling outperforms liquid cooling such as water cooling. It is thought that liquid cooling has a more corrosive effect on parts of the plasma source than the gas cooling, thus causing larger temperature variations.

Also, the relatively short electrical conductor appears to beneficially influence the quality of the disinfecting and/or sterilizing gas mixture. Although not fully understood, it is believed that the relatively short electrical conductor has a beneficial effect on electromagnetic compatibility (EMC) and/or reduces electrical impedance of the system, which can be beneficial. The reduced variations in temperature can contribute to more stable production of desired disinfecting and/or sterilizing components in the gas mixture.

Optionally, the forced gas cooling system includes a temperature control system for controlling the temperature of the plasma and/or the ionization chamber and/or the tubular portion. The temperature control system can include a temperature sensor and a controller.

Optionally, the forced gas cooling system includes a detector for detecting malfunction of the cooling system and is arranged for shutting down or reducing power of the high voltage source when a malfunction is detected. Hence overheating of the plasma source in case of a malfunction of the cooling system can be avoided.

Optionally, the electrical conductor is less than 50cm long, preferably less than 30 cm long, more preferably less than 20 cm long.

Optionally, the plasma source includes a first end cap including the inflow port and closing the dielectric tubular portion at a first end. Optionally, the plasma source includes a second end cap including the outflow port and closing the dielectric tubular portion at a second end opposite the first end. The end caps provide an effective and mechanically simple way of providing the inflow port and/or outflow port to the dielectric tubular portion. Preferably, the first and/or second end cap is made of an electrically insulating material.

Optionally, the dielectric tube includes a wall of quartz or a glass, such as a borosilicate glass, such as Pyrex^{®} or Duran^{®}.

Optionally, the plasma source includes a, e.g. metal, housing. Optionally, the ionization chamber, the high voltage source, and at least part of the forced gas cooling system are included in the housing. Hence a plasma source can be provided that can easily be installed and/or replaced. Also, when the housing is a metal housing EMC can easily be obtained.

The gas or gas mixture fed to the inflow port can be a humidified gas or gas mixture, such as humidified air. The gas or gas mixture can have a predetermined specific humidity, e.g. of between 2 to 25 grams of water vapor per kilogram of gas, such as about 10 grams of water vapor per kilogram of gas. The gas or gas mixture can be humidified e.g. as described in copending patent application NL2025110, incorporated herein by reference.

According to a second aspect is provided a sterilization apparatus for sterilizing medical instruments, including a plasma source as described hereinabove.

According to a third aspect is provided a method for generating a disinfecting gas mixture. The method includes feeding a gas or gas mixture through a dielectric tubular portion having a first electrode positioned inside the dielectric tubular portion, and a second electrode positioned outside the dielectric tubular portion. The method includes applying a high voltage difference between the first and second electrodes. The method includes
cooling the dielectric tubular portion using a forced gas cooling system, wherein the forced gas cooling system comprises an elongate funnel having two sidewalls tapering towards the dielectric tubular portion and extending along a longitudinal axis of the dielectric tubular portion, wherein at least one fan blows the cooling gas flow into a wide end of the funnel, causing a concentrated and/or accelerated flow of the cooling gas to exit a narrow end of the funnel to impinge one side of the tubular portion in a direction orthogonal to the longitudinal axis, the impinging gas thereafter flowing around the tubular portion to another side of the tubular portion, opposite said one side, thereafter being directed orthogonally away from the tubular portion.

Optionally, the method includes providing the high voltage to the first or second electrode via a relatively short electrical conductor.

It will be appreciated that any of the aspects, features and options described in view of the plasma source apply equally to the sterilization apparatus and the method, and vice versa. It will also be clear that any one or more of the above aspects, features and options can be combined.

### BRIEF DESCRIPTION OF THE DRAWINGS

Embodiments of the present invention will now be described in detail with reference to the accompanying drawings in which:
Figure 1 shows a schematic representation of a plasma source;
Figure 2A shows a schematic representation of a plasma source;
Figure 2B illustrates a cross-section view of a forced gas cooling system.
Figure 3 shows a schematic representation of a sterilizing apparatus; and
Figure 4 shows a schematic representation of a method.

### DETAILED DESCRIPTION

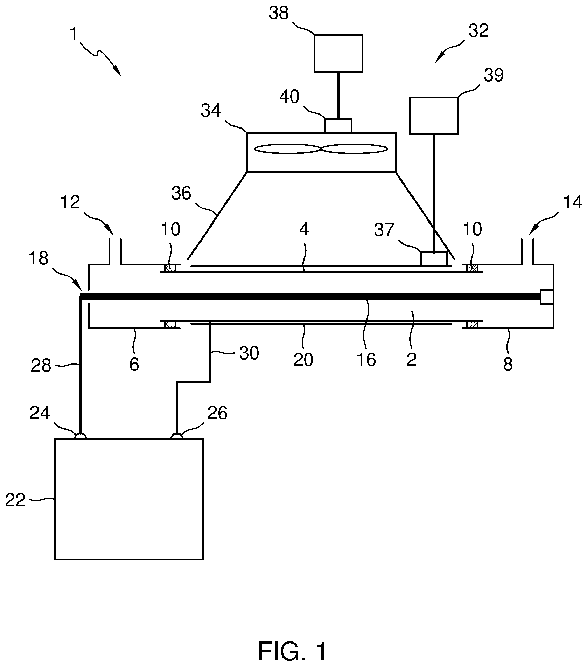

Figure 1 shows a schematic representation of a plasma source 1 for generating a disinfecting and/or sterilizing gas mixture. The plasma source 1 includes an ionization chamber 2. The ionization chamber 2 here is bounded by walls. A first wall is formed by a dielectric tubular portion 4. The dielectric tube can e.g. include, or be, a glass tube, e.g. made of quartz or glass, such a borosilicate glass, such as Pyrex^{®} or Duran^{®}.

In this example, a second wall is formed by a first end cap 6 closing the dielectric tubular portion 4 at a first end. In this example a third wall is formed by a second end cap 8 closing the dielectric tubular portion 4 at a second end opposite the first end. Here the end caps 6, 8 are connected to the tubular portion 4 in a gastight manner. Here a seal, such as an O-ring 10 is provided between the end cap 6, 8 and the tubular portion 4.

The ionization chamber 2 includes an inflow port 12 for feeding a gas or gas mixture into the chamber 2. Here, the inflow port is positioned at the first end of the tubular portion. In this example, the inflow port 12 forms part of the first end cap 6. The ionization chamber 2 includes an outflow port 14 for exhausting the sterilizing gas out of the chamber 2. Here, the outflow port 14 is positioned at the second end of the tubular portion 4. In this example, the outflow port 14 forms part of the second end cap 8.

The ionization chamber 2 includes a first electrode 16. The first electrode 16 is positioned inside the dielectric tubular portion 2. Here, the first electrode extends longitudinally within the tubular portion 2, here along the axis of the tubular portion 2. The first electrode 16 in this example is elongate, such as rod shaped. Here the first electrode 16 has a thicker rod diameter at the area where plasma is to be generated. In this example, the chamber 2 includes an electric feedthrough 18 forming an electrical connection from outside the chamber to the first electrode 16 inside the chamber 2. Here the feedthrough is positioned in the first end cap 6. In Fig.1 a gap is drawn between the feedthrough 18 and the first end cap 6 for clarity. It will be appreciated that in reality the feedthrough forms a gas tight electrical connection from outside the chamber 2 to inside the chamber 2. In this example both the first end cap 6 and the second end cap 8 include retainers for retaining the first electrode in its position.

The ionization chamber 2 includes a second electrode 20. The second electrode 20 is positioned outside the dielectric tubular portion 4. The second electrode 20 can be formed on an outer surface of the tubular portion 4. The second electrode can be a separate part, such as a metal sheet positioned on the outer surface of the tubular portion 4, such as in intimate contact with the outer surface of the tubular portion 4. In this example, the second electrode 20 is formed as a conductive layer coated onto the outer surface of the tubular portion 4, such as a metallic layer (plasma) deposited onto the outer surface. In Fig.1 a gap is drawn between the second electrode 20 and the tubular portion 4 for clarity. It will be appreciated that in reality the second electrode 20 and the tubular portion are in contact with each other.

The plasma source 1 includes a high voltage source 22. The high voltage source 22 is arranged for supplying a high voltage difference between two output terminals 24, 26. In this example, the first output terminal 24 is a high voltage output terminal, and the second output terminal 26 is connected or connectable to electrical ground. The high voltage supplied at the first output terminal 24 can be a positive high voltage or a negative high voltage. In this example, a first electrical conductor 28 connects the first output terminal 24 to the first electrode 16. Here a second electrical conductor 30 connects the second output terminal 26 to the second electrode 20. It will be appreciated that it is also possible that the second output terminal 26 and the second electrode 20 are both connected to electrical ground. In such case a dedicated second electrical conductor 30 in the form of a lead wire may be omitted.

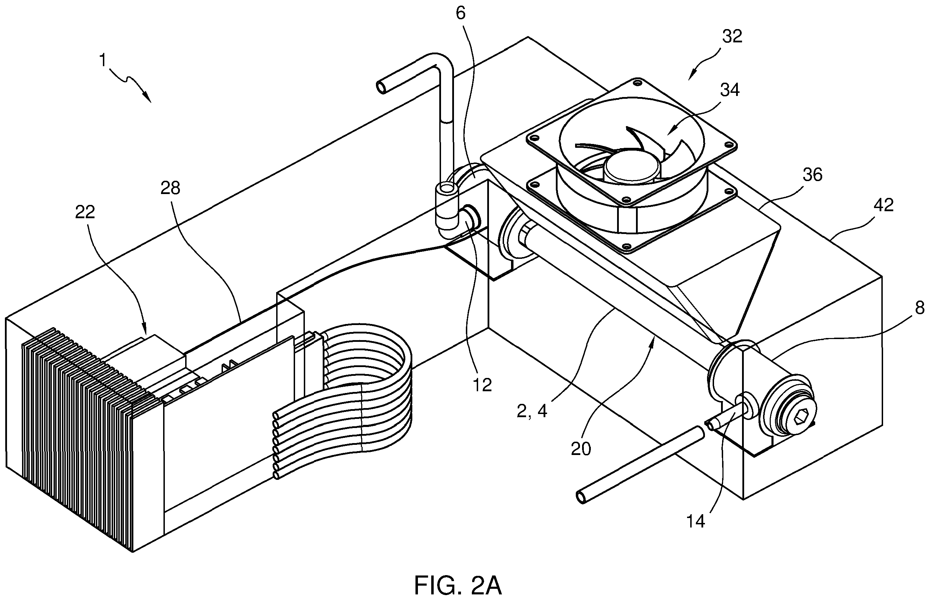

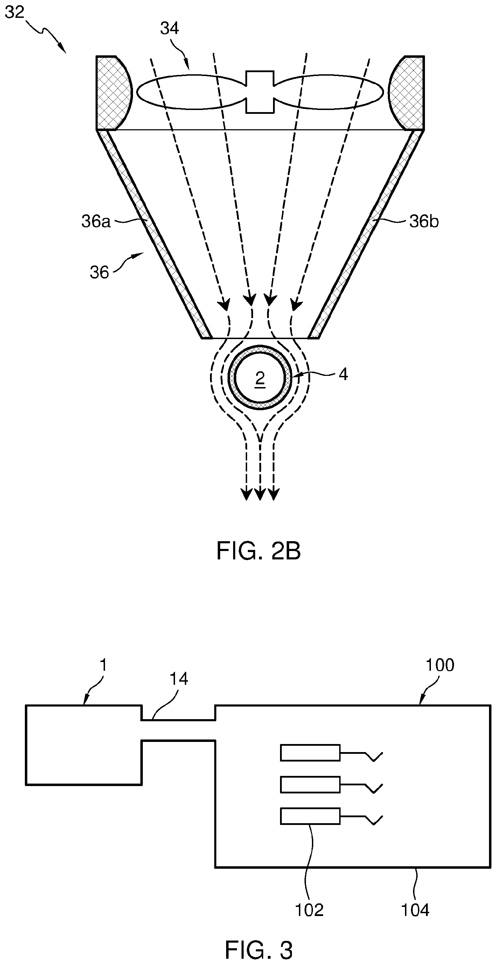

The plasma source 1 includes a forced gas cooling system 32. The forced gas cooling system 32 in Fig. 1 includes a fan 34. The forced gas cooling system 32 in Fig. 1 further includes a guide 36 for guiding cooling gas, towards the chamber 2. The guide 36 includes a funnel. Fig. 2A shows an exemplary three-dimensional view of a plasma source 1. Fig. 2B illustrates a cross-section view of the forced gas cooling system 32 acting on the ionization chamber 2. In Figs. 2A and 2B a cross section of the guide 36 tapers towards the chamber 2. As shown in FIG 2A, the guide is formed by an elongate guide or funnel extending along a length of the tubular portion 4, e.g. between the end caps 6 and 8. As shown, a length of the elongate guide or funnel may similar or the same as a length of the tubular portion 4, e.g. within ±20%. In this way the cooling can take place along substantially the whole length. The forced gas cooling system comprises an elongate funnel 36 having two sidewalls 36a,36b tapering towards the dielectric tubular portion 4 and extending along the longitudinal axis. As shown, the funnel is arranged between the fan 34 and the dielectric tubular portion 4. Also multiple fans (not shown) can be arranged along the wide end of the funnel, e.g. arranged side by side along the longitudinal axis. The cooling system is configured to force the cooling gas flow by the fan into a wide end of the funnel 36, wherein the gas flow exits a narrow end of the funnel to impinge one side of the tubular portion orthogonal to the longitudinal axis. For example, the wide end of the funnel 36 is wider than the narrow end by at least a factor 1.2 (20%), 1.5 (50%), 2 (twice as wide), or more. Accordingly, a relatively high gas flow can be easily established at or near the tubular portion 4. For example, the gas flow can be guided around the tube (e.g. in a split gas flow) from said one side to an opposite side of the tubular portion and carrying heat away from the ionization chamber 2. Additionally to the shown gas flow, also other or further gas flows can be established, e.g. by a fan directing a gas flow away from the ionization chamber 2. For example, one or more fans can be arranged for forcing a gas flow into and/or out of the housing 42.

In the example of Fig. 2A the plasma source 1 includes a housing 42, such as a metallic housing, e.g. shielding electromagnetic radiation. In this example, the ionization chamber 2, the high voltage source 22, and at least part of the forced gas cooling system 32 are included in the housing 42. In this particular example, the fan 34 forms part of the housing 42. In Fig. 2A the housing 42 is shown as transparent for clarity.

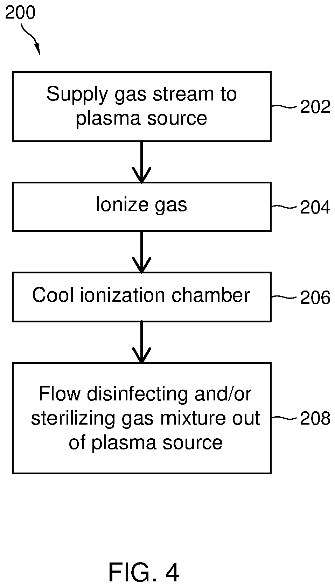

The plasma source 1 as described thus far can be used as follows in a method 200 for generating a disinfecting and/or sterilizing gas mixture, also see Fig. 4. A gas or gas mixture, such as air, e.g. air having a predetermined specific humidity, is fed 202 into the ionization chamber 2 via the inflow port 12. In the ionization chamber 2 a plasma is generated 204 by applying a high voltage difference between the first electrode 16 and the second electrode 20. As a result the gas or gas mixture flowing through the ionization chamber 2 is at least partially ionized to form the disinfecting and/or sterilizing gas mixture. The disinfecting and/or sterilizing gas mixture flows 208 out of the ionization chamber via the outflow port 14.

During ionization, i.e. during generation of the plasma, the ionization chamber is cooled 206 using the forced gas cooling system 32. In this example, the fan 34 generates a stream of air blowing towards the ionization chamber 2. Here, the stream of air is directed towards the ionization chamber, e.g. towards the tubular portion 4, by the guides 36. Here, the stream of air is directed in a direction substantially orthogonal to a longitudinal axis of the tubular portion 4, here perpendicular to the longitudinal axis of the tubular portion 4. In principle, the forced gas cooling system can also be arranged for forcing a cooling gas flow onto the dielectric tubular portion in a direction substantially parallel to a longitudinal axis of the tubular portion. However, by blowing the gas orthogonal to the tubular portion, heat can be more quickly dissipated and/or more homogenous. For example, the orthogonally flowing heated gas can immediately leave the vicinity of the tubular portion compared to heated gas flowing along a length of the tube. For example, the orthogonally flowing gas can have a substantially uniform temperature along the length of the tube compared to a gas flowing heating up as it flows along the length of the tube. By using the forced gas cooling, the temperature of the ionization chamber, and the gas or gas mixture therein, can be maintained substantially constant. It has been found that this has a beneficial effect on the quality of the disinfecting and/or sterilizing gas mixture.

It has also been found that providing the first electrical conductor 28, providing the high voltage, having a relatively short length appears to beneficially influence the quality of the disinfecting and/or sterilizing gas mixture. Although not fully understood, it is believed that the relatively short electrical conductor reduces variations in supply voltage which reduces the variations in temperature. The reduced variations in temperature can contribute to more stable production of desired disinfecting and/or sterilizing components in the gas mixture. Here the relatively short length is a length of 50 cm or less, preferably 30 cm or less, more preferably 20 cm or less. It will be appreciated that it is possible that the first output terminal 24 is directly connected to the first electrode 16. Then the length of the first electrical conductor 28 is zero. The second output terminal 26 can also be directly connected to the second electrode 20. Then the length of the second electrical conductor 30 is zero.

The forced gas cooling system 32 cooling the chamber 2, such as cooling the outside of the tubular portion 4, while the plasma is being generated, can cause a gradient in temperature in the ionization chamber 2.

The forced gas cooling system 32 can include a temperature control system 37 for controlling the temperature of the plasma and/or the ionization chamber 2 and/or the tubular portion 4. The temperature control system 37 can include a temperature sensor 39. The sensor 39 can e.g. be mounted inside the ionization chamber 2, to an inner surface or outer surface of the tubular portion, or in the proximity of the tubular portion 4 outside the chamber 2. Alternatively, or additionally, a temperature sensor 39 can be placed in the gas stream output from the ionization chamber 2. Controlling the temperature of the plasma, e.g. by controlling the temperature of the ionization chamber 2 or the tubular portion 4, can provide two advantages. The controlled temperature of the plasma aids in beneficially influencing temperature stability of the plasma source. Also, by adjusting the setpoint of the controlled temperature of the plasma a quality of the disinfecting and/or sterilizing gas mixture, e.g. a composition of the disinfecting and/or sterilizing gas mixture, can be selected.

The forced gas cooling system 32 can include a detection system 38 for detecting malfunction of the cooling system. The detection system 38 can be arranged for shutting down or reducing power of the high voltage source 22 when a malfunction of the cooling system 32 is detected. Hence overheating of the plasma source in case of a malfunction of the cooling system 32 can be avoided. The detection system can include a detector 40 for detecting malfunction of the cooling system 32. The detector 40 can include a gas flow sensor for monitoring flowing of the cooling gas. The detector 40 can include a current sensor for sensing a motor current of the fan 34. The detector 40 can include a temperature sensor, e.g. the sensor 39, for sensing a temperature of the plasma source 1, e.g. a temperature of the chamber 2, the tubular portion 4 and/or the housing 42. The detector 40 can e.g. be a thermal switch.

Fig. 3 shows an example of a sterilization apparatus 100 for sterilizing medical instruments 102, such as dental instruments. The sterilization apparatus 100 includes a plasma source 1 as described in view of Fig. 1 and Fig. 2. The sterilization apparatus 100 includes a sterilization chamber 104. The disinfecting and/or sterilizing gas mixture is fed from the plasma source 1 into the sterilization chamber 104, towards the instruments 102 included in the chamber 104.

Herein, the invention is described with reference to specific examples of embodiments of the invention. It will, however, be evident that various modifications and changes may be made.

In the example of Fig. 1 the first electrode is connected to high voltage and the second electrode is connected to electrical ground. It will be appreciated that it is also possible that the first electrode is connected to electrical ground and the second electrode is connected to high voltage. It is also possible that both the first and second electrodes are connected to high voltage, for example one to positive high voltage and the other to negative high voltage. Preferably the electrical conductor(s) providing the high voltage to the first or second electrode has the relatively short length of 50 cm or less, preferably 30 cm or less, more preferably 20 cm or less.

Preferably, the high voltage source as described herein is configured to generate a high voltage and/or current within a relatively short time span, e.g. within less than fifty milliseconds after startup (e.g. starting at zero volt/amp), preferably less than twenty milliseconds. More preferably, the voltage and/or current is ramped up after startup with an initial overshoot exceeding the nominal operating voltage thereafter, e.g. by at least 10%, to initiate the plasma creation. For example, the high voltage source is powered by a power supply adapted to allow such rapid startup. The inventors find that these setting may lead to a more stable and/or reliable plasma. In the claims, any reference signs placed between parentheses shall not be construed as limiting the claim. The word 'comprising' does not exclude the presence of other features or steps than those listed in a claim. Furthermore, the words 'a' and 'an' shall not be construed as limited to 'only one', but instead are used to mean `at least one', and do not exclude a plurality.

## Claims

1. A plasma source (1) for generating a disinfecting and/or sterilizing gas mixture, including:
- an ionization chamber (2) having:
- a dielectric tubular portion (4),
- an inflow port (12) for feeding a gas or gas mixture into the chamber,
- an outflow port (14) for exhausting the disinfecting and/or sterilizing gas mixture out of the chamber,
- a first electrode (16) positioned inside the dielectric tubular portion (4), and
- a second electrode (20) positioned outside the dielectric tubular portion (4);
- a high voltage source (22) having a high voltage output terminal (24, 26), wherein an electrical conductor (28, 30) connects the output terminal (24, 26) to the first or second electrode (16, 20); and
- a forced gas cooling system (32) for cooling the ionization chamber (2), **characterized in that**,
the forced gas cooling system is arranged for forcing a cooling gas flow onto the dielectric tubular portion (4) in a direction substantially orthogonal to a longitudinal axis of the tubular portion,
wherein the forced gas cooling system comprises an elongate funnel (36) having two sidewalls (36a, 36b) tapering towards the dielectric tubular portion (4) and extending along the longitudinal axis, said forced gas cooling system (32) further comprising at least one fan (34), wherein the funnel (36) is arranged between at least one fan (34) and the dielectric tubular portion (4) and configured to force the cooling gas flow by the fan into the funnel (36) towards its narrow end where the two sidewalls converge, wherein said narrow end has an elongate exit opening disposed adjacent the dielectric tubular portion and extending along the longitudinal axis, wherein the exit opening is arranged facing the dielectric tubular portion and configured to force the cooling gas flow exiting the opening to flow onto one side of the tubular portion in the direction substantially orthogonal to the longitudinal axis.

2. The plasma source (1) of claim 1, wherein the forced gas cooling system includes a detector (40) for detecting malfunction of the cooling system (32) and is arranged for shutting down or reducing power of the high voltage source when a malfunction is detected.

3. The plasma source (1) of claim 1 or 2, wherein the electrical conductor (28, 30) is less than 50cm long, preferably less than 30 cm long, more preferably less than 20 cm long.

4. The plasma source (1) of any of claims 1-3, wherein the second electrode (20) is an electrically conducting layer deposited onto an outer surface of the dielectric tubular portion (4).

5. The plasma source (1) of any of claims 1-4, wherein the second electrode (20) is connected to electrical ground.

6. The plasma source (1) of any of claims 1-5, including a first end cap (6) including the inflow port (12) and closing the dielectric tubular portion (4) at a first end, and a second end cap (8) including the outflow port (14) and closing the dielectric tubular portion (4) at a second end opposite the first end.

7. The plasma source (1) of claim 6, wherein the first and/or second end cap (6, 8) is made of an electrically insulating material.

8. The plasma source (1) of any of claims 1-7, wherein the dielectric tubular portion (4) includes a wall of quartz or a glass, such as a borosilicate glass.

9. The plasma source (1) of any of claims 1-8, including a housing (42), wherein the ionization chamber (2), the high voltage source (22), and at least part of the forced gas cooling system (32) are included in the housing.

10. The plasma source (1) of any of claims 1-9, wherein the high voltage source (22) is configured to generate a high voltage and/or current within a relatively short time span of less than fifty milliseconds after startup, preferably less than twenty milliseconds, wherein the high voltage source is configured to ramp up the high voltage and/or current at startup with an initial overshoot to initiate plasma creation, thereafter decreasing to a nominal operating value for maintaining the plasma creation, wherein the initial overshoot is at least ten percent higher than the nominal operating value.

11. A sterilization apparatus (100) for sterilizing medical instruments, **characterized in that**,
the sterilization apparatus (100) includes a plasma source (1) according to any of claims 1-10.

12. A method for generating a disinfecting and/or sterilizing gas mixture, including:
- feeding a gas or gas mixture through a dielectric tubular portion (4) having a first electrode (16) positioned inside the dielectric tubular portion, and a second electrode (20) positioned outside the dielectric tubular portion;
- applying a high voltage difference between the first and second electrodes (16, 20), **characterized in that**,
the method further includes:
- cooling the dielectric tubular portion using a forced gas cooling system (32), wherein the forced gas cooling system comprises an elongate funnel (36) having two sidewalls (36A, 36B) tapering towards the dielectric tubular portion (4) and extending along a longitudinal axis of the dielectric tubular portion, wherein at least one fan (34) blows the cooling gas flow into a wide end of the funnel, causing a concentrated and/or accelerated flow of the cooling gas to exit a narrow end of the funnel to impinge one side of the tubular portion (4) in a direction orthogonal to the longitudinal axis, the impinging gas thereafter flowing around the tubular portion to another side of the tubular portion, opposite said one side, thereafter being directed orthogonally away from the tubular portion.

## Patentansprüche

1. Plasmaquelle (1) zum Erzeugen eines desinfizierenden und/oder sterilisierenden Gasgemischs, beinhaltend:
- eine Ionisationskammer (2), aufweisend:
- einen dielektrischen rohrförmigen Abschnitt (4),
- einen Zuflussstutzen (12) zum Einspeisen eines Gases oder Gasgemischs in die Kammer,
- einen Ausflussstutzen (14) zum Entlüften des desinfizierenden und/oder sterilisierenden Gasgemischs aus der Kammer,
- eine erste Elektrode (16), die innerhalb des dielektrischen rohrförmigen Abschnitts (4) positioniert ist, und
- eine zweite Elektrode (20), die außerhalb des dielektrischen rohrförmigen Abschnitts (4) positioniert ist;
- eine Hochspannungsquelle (22) mit einer Hochspannungsausgangsklemme (24, 26), wobei ein elektrischer Leiter (28, 30) die Ausgangsklemme (24, 26) mit der ersten oder der zweiten Elektrode (16, 20) verbindet; und
- ein erzwungenes Gaskühlsystem (32) zum Kühlen der Ionisationskammer (2), **dadurch gekennzeichnet, dass**
das erzwungene Gaskühlsystem zum Zwingen eines Kühlgasstroms auf den dielektrischen rohrförmigen Abschnitt (4) in einer Richtung, die im Wesentlichen orthogonal zu einer Längsachse des rohrförmigen Abschnitts ist, eingerichtet ist,
wobei das erzwungene Gaskühlsystem einen länglichen Trichter (36) mit zwei Seitenwänden (36a, 36b) umfasst, die sich zu dem dielektrischen rohrförmigen Abschnitt (4) hin verjüngen und sich entlang der Längsachse erstrecken, wobei das erzwungene Gaskühlsystem (32) weiterhin mindestens ein Gebläse (34) umfasst, wobei der Trichter (36) zwischen mindestens einem Gebläse (34) und dem dielektrischen rohrförmigen Abschnitt (4) eingerichtet ist, und dazu konfiguriert ist, den Kühlgasstrom durch das Gebläse in den Trichter (36) zu seinem schmalen Ende hin zu zwingen, wo die zwei Seitenwände zusammenlaufen, wobei das schmale Ende eine längliche Austrittsöffnung aufweist, die angrenzend an den dielektrischen rohrförmigen Abschnitt angeordnet ist und sich entlang der Längsachse erstreckt, wobei die Austrittsöffnung dem dielektrischen rohrförmigen Abschnitt zugewandt eingerichtet ist und dazu konfiguriert ist, den Kühlgasstrom, der aus der Öffnung austritt, zu zwingen, auf eine Seite des rohrförmigen Abschnitts in der Richtung, die im Wesentlichen orthogonal zu der Längsachse ist, zu strömen.

2. Plasmaquelle (1) nach Anspruch 1, wobei das erzwungene Gaskühlsystem einen Detektor (40) zum Erkennen einer Fehlfunktion des Kühlsystems (32) beinhaltet und zum Herunterfahren oder Verringern der Energie der Hochspannungsquelle eingerichtet ist, wenn eine Fehlfunktion erkannt wird.

3. Plasmaquelle (1) nach Anspruch 1 oder 2, wobei der elektrische Leiter (28, 30) weniger als 50 cm lang, vorzugsweise weniger als 30 cm lang, mehr bevorzugt weniger als 20 cm lang ist.

4. Plasmaquelle (1) nach einem der Ansprüche 1 bis 3, wobei die zweite Elektrode (20) eine elektrisch leitende Schicht ist, die auf einer Außenfläche des dielektrischen rohrförmigen Abschnitts (4) abgeschieden ist.

5. Plasmaquelle (1) nach einem der Ansprüche 1 bis 4, wobei die zweite Elektrode (20) mit elektrischer Masse verbunden ist.

6. Plasmaquelle (1) nach einem der Ansprüche 1 bis 5, beinhaltend eine erste Endkappe (6), beinhaltend den Zuflussstutzen (12) und den dielektrischen rohrförmigen Abschnitt (4) an einem ersten Ende schließend, und eine zweite Endkappe (8), beinhaltend den Ausflussstutzen (14) und den dielektrischen rohrförmigen Abschnitt (4) an einem zweiten Ende, das entgegengesetzt zu dem ersten Ende ist, schließend.

7. Plasmaquelle (1) nach Anspruch 6, wobei die erste und/oder die zweite Endkappe (6, 8) aus einem elektrisch isolierenden Material hergestellt sind.

8. Plasmaquelle (1) nach einem der Ansprüche 1 bis 7, wobei der dielektrische rohrförmige Abschnitt (4) eine Wand aus Quarz oder einem Glas, wie Borosilikatglas, beinhaltet.

9. Plasmaquelle (1) nach einem der Ansprüche 1 bis 8, beinhaltend ein Gehäuse (42), wobei die Ionisationskammer (2), die Hochspannungsquelle (22) und mindestens ein Teil des erzwungenen Gaskühlsystems (32) in dem Gehäuse enthalten sind.

10. Plasmaquelle (1) nach einem der Ansprüche 1 bis 9, wobei die Hochspannungsquelle (22) dazu konfiguriert ist, eine hohe Spannung und/oder Stromstärke innerhalb einer relativ kurzen Zeitspanne von weniger als fünfzig Millisekunden nach dem Hochfahren, vorzugsweise weniger als zwanzig Millisekunden zu erzeugen, wobei die Hochspannungsquelle dazu konfiguriert ist, die hohe Spannung und/oder Stromstärke beim Hochfahren mit einem anfänglichen Überschwingen zu beschleunigen, um eine Plasmaerzeugung zu initiieren, wonach auf einen Nennbetriebswert zum Aufrechterhalten der Plasmaerzeugung verringert wird, wobei das anfängliche Überschwingen um mindestens zehn Prozent höher als der Nennbetriebswert ist.

11. Sterilisationsvorrichtung (100) zum Sterilisieren von medizinischen Instrumenten, **dadurch gekennzeichnet, dass**
die Sterilisationsvorrichtung (100) eine Plasmaquelle (1) nach einem der Ansprüche 1 bis 10 beinhaltet.

12. Verfahren zum Erzeugen eines desinfizierenden und/oder sterilisierenden Gasgemischs, beinhaltend:
- Einspeisen eines Gases oder Gasgemischs durch einen dielektrischen rohrförmigen Abschnitt (4) mit einer ersten Elektrode (16), die innerhalb des dielektrischen rohrförmigen Abschnitts positioniert ist, und einer zweiten Elektrode (20), die außerhalb des dielektrischen rohrförmigen Abschnitts positioniert ist;
- Anwenden einer Hochspannungsdifferenz zwischen der ersten und der zweiten Elektrode (16, 20), **dadurch gekennzeichnet, dass**
das Verfahren weiterhin beinhaltet:
- Kühlen des dielektrischen rohrförmigen Abschnitts unter Verwendung eines erzwungenen Gaskühlsystems (32), wobei das erzwungene Gaskühlsystem einen länglichen Trichter (36) mit zwei Seitenwänden (36A, 36B) umfasst, die sich zu dem dielektrischen rohrförmigen Abschnitt (4) hin verjüngen und sich entlang einer Längsachse des dielektrischen rohrförmigen Abschnitts erstrecken, wobei mindestens ein Gebläse (34) den Kühlgasstrom in ein breites Ende des Trichters bläst, was einen konzentrierten und/oder beschleunigten Strom des Kühlgases bewirkt, um aus einem schmalen Ende des Trichters auszutreten, um auf eine Seite des rohrförmigen Abschnitts (4) in einer Richtung, die orthogonal zu der Längsachse ist, aufzuprallen, wobei das aufprallende Gas danach um den rohrförmigen Abschnitt zu einer anderen Seite des rohrförmigen Abschnitts, die entgegengesetzt zu der einen Seite ist, strömt, wonach er orthogonal von dem rohrförmigen Abschnitt weg geleitet wird.

## Revendications

1. Source de plasma (1) pour production d'un mélange gazeux désinfectant et/ou stérilisant, comportant :
- une chambre d'ionisation (2) présentant :
- une partie tubulaire diélectrique (4),
- un orifice d'entrée (12) permettant d'envoyer un gaz ou un mélange gazeux dans la chambre,
- un orifice de sortie (14) permettant d'évacuer hors de la chambre le mélange gazeux désinfectant et/ou stérilisant,
- une première électrode (16) placée à l'intérieur de la partie tubulaire diélectrique (4),
- et une deuxième électrode (20) placée à l'extérieur de la partie tubulaire diélectrique (4),
- une source de haute tension (22) dotée d'une borne de sortie haute tension (24, 26), étant entendu qu'un conducteur électrique (28,30) relie la borne de sortie (24, 26) à la première ou à la deuxième électrode (16, 20),
- et un système (32) de refroidissement par gaz forcé, permettant de refroidir la chambre d'ionisation (2),
**caractérisée en ce que**
le système de refroidissement par gaz forcé est disposé pour envoyer de force un courant de gaz réfrigérant sur la partie tubulaire diélectrique (4), dans une direction pratiquement perpendiculaire à l'axe longitudinal de la partie tubulaire,
lequel système de refroidissement par gaz forcé comprend un entonnoir allongé (36) doté de deux parois latérales (36a, 36b) qui se rapprochent en allant vers la partie tubulaire diélectrique et s'étendent le long de l'axe longitudinal, lequel système (32) de refroidissement par gaz forcé comprend en outre au moins un ventilateur (34), l'entonnoir (36) étant disposé entre le ventilateur (34) au nombre d'au moins un et la partie tubulaire diélectrique (4) et configuré de manière à ce qu'un courant de gaz réfrigérant soit envoyé de force par le ventilateur dans l'entonnoir (36) en direction de son extrémité étroite où convergent les deux parois latérales, laquelle extrémité étroite présente un orifice de sortie allongé, situé en position adjacente à la partie tubulaire diélectrique et s'étendant le long de l'axe longitudinal, lequel orifice de sortie est disposé en face de la partie tubulaire diélectrique et configuré de manière à ce que le courant de gaz réfrigérant qui sort de l'orifice soit envoyé de force sur un côté de la partie tubulaire, dans une direction pratiquement perpendiculaire à l'axe longitudinal.

2. Source de plasma (1) conforme à la revendication 1, dans laquelle le système de refroidissement par gaz forcé comporte un détecteur (40) servant à détecter un dysfonctionnement du système de refroidissement (32) et conçu pour débrancher la source de haute tension ou en réduire la puissance quand un dysfonctionnement est détecté.

3. Source de plasma (1) conforme à la revendication 1 ou 2, dans laquelle le conducteur électrique (28, 30) est long de moins de 50 cm, de préférence long de moins de 30 cm, et mieux encore long de moins de 20 cm.

4. Source de plasma (1) conforme à l'une des revendications 1 à 3, dans laquelle la deuxième électrode (20) est une couche électro-conductrice déposée sur une surface externe de la partie tubulaire diélectrique (4).

5. Source de plasma (1) conforme à l'une des revendications 1 à 4, dans laquelle la deuxième électrode est électriquement connectée à la terre.

6. Source de plasma (1) conforme à l'une des revendications 1 à 5, comportant un premier capuchon (6) d'extrémité, qui comprend l'orifice d'entrée (12) et qui ferme la partie tubulaire diélectrique (4) au niveau d'une première extrémité, et un deuxième capuchon (8) d'extrémité, qui comprend l'orifice de sortie (14) et qui ferme la partie tubulaire diélectrique (4) au niveau d'une deuxième extrémité située à l'opposé de la première extrémité.

7. Source de plasma (1) conforme à l'une des revendications 1 à 6, dans laquelle les premier et/ou deuxième capuchons (6,8) d'extrémité sont faits d'un matériau électriquement isolant.

8. Source de plasma (1) conforme à l'une des revendications 1 à 7, dans laquelle la partie tubulaire diélectrique (4) comprend une paroi en quartz ou en un verre, comme un verre de borosilicate.

9. Source de plasma (1) conforme à l'une des revendications 1 à 8, qui comprend un logement (42) et dans laquelle la chambre d'ionisation (2), la source de haute tension (22) et au moins une partie du système de refroidissement par gaz forcé (32) sont disposés dans le logement.

10. Source de plasma (1) conforme à l'une des revendications 1 à 9, dans laquelle la source de haute tension (22) est configurée de manière à générer une tension élevée et/ou un courant fort en un laps de temps relativement court, de moins de 50 millisecondes et de préférence de moins de 20 millisecondes après démarrage, étant entendu que la source de haute tension est configurée pour faire augmenter très brutalement la tension et/ou le courant à une valeur élevée dès le démarrage tout en provoquant une surcharge initiale pour amorcer la formation d'un plasma, et pour les faire ensuite diminuer jusqu'à une valeur nominale de fonctionnement pour entretenir la formation du plasma, laquelle surcharge initiale est d'au moins dix pour cent plus élevée que la valeur nominale de fonctionnement.

11. Appareillage de stérilisation (100) destiné à la stérilisation d'instruments médicaux, **caractérisé en ce que** l'appareillage de stérilisation (100) comprend une source de plasma (1) conforme à l'une des revendications 1 à 10.

12. Procédé de production d'un mélange gazeux désinfectant et/ou stérilisant, comportant :
- le fait d'envoyer un gaz ou un mélange gazeux traverser une partie tubulaire diélectrique (4) dotée d'une première électrode (16) placée à l'intérieur de la partie tubulaire diélectrique et d'une deuxième électrode (20) placée à l'extérieur de la partie tubulaire diélectrique,
- et le fait d'appliquer une différence de potentiel élevée entre les première et deuxième électrodes (16, 20),
lequel procédé est **caractérisé en ce qu'**il comporte en outre
- le fait de refroidir la partie tubulaire diélectrique à l'aide d'un système (32) de refroidissement par gaz forcé, lequel système de refroidissement par gaz forcé comprend un entonnoir allongé (36) doté de deux parois latérales (36A, 36B) qui se rapprochent en allant vers la partie tubulaire diélectrique (4) et s'étendent le long de l'axe longitudinal de la partie tubulaire diélectrique, et dans lequel système au moins un ventilateur (34) propulse le courant de gaz réfrigérant dans l'extrémité évasée de l'entonnoir, ce qui fait qu'un courant concentré et/ou accéléré de gaz réfrigérant sort de l'entonnoir par l'extrémité étroite de celui-ci pour aller impacter un premier côté de la partie tubulaire diélectrique (4) dans une direction pratiquement perpendiculaire à l'axe longitudinal, lequel gaz impactant circule ensuite autour de la partie tubulaire jusqu'à un autre côté de la partie tubulaire, à l'opposé dudit premier côté, pour s'éloigner ensuite de la partie tubulaire dans une direction perpendiculaire à celle-ci.
